Europäisches Patentamt

European Patent Office

Office européen des brevets

(11) Publication number: **0 169 895**
**B1**

# (12) EUROPEAN PATENT SPECIFICATION

(45) Date of publication of patent specification: **09.08.89**

(21) Application number: **85901100.9**

(22) Date of filing: **27.01.85**

(86) International application number:
**PCT/RO85/00001**

(87) International publication number:
**WO 85/03229 01.08.85 Gazette 85/17**

(51) Int. Cl.⁴: **A 61 K 35/78**

(54) MEDICINAL VEGETABLE COMPOSITION FOR TREATING SOME HEPATIC AND BILIARY DISEASES.

(30) Priority: **27.01.84 RO 113423**

(43) Date of publication of application:
**05.02.86 Bulletin 86/06**

(45) Publication of the grant of the patent:
**09.08.89 Bulletin 89/32**

(84) Designated Contracting States:
**FR**

(56) References cited:
**none**

The file contains technical information
submitted after the application was filed and
not included in this specification

(73) Proprietor: **DIRECTIA SANITARA A JUDETULUI NEAMT**
**Laboratoarele Plantavorel Str. Bd. Traian Nr. 1**
**Piatra Neamt, COD 5600 (RO)**

(72) Inventor: **ELTHES, Ludovic, Ladislau**
**Str. Bela Breiner Nr. 72**
**Oradea COD 3600 (RO)**
Inventor: **ELTHES, Aristina**
**Str. Bela Breiner Nr. 72**
**Oradea COD 3600 (RO)**

(74) Representative: **Chameroy, Claude et al**
**c/o Cabinet Malemont 42, avenue du Président Wilson**
**F-75116 Paris (FR)**

Courier Press, Leamington Spa, England.

## Description

The present invention refers to a medicinal herbal composition for treating functional or organic hepatic and biliary diseases, aiming at restoring the basic hepatic function.

Some drugs such as "Essentiale", "Purinor-"—"Produse farmaceutice folosite in practica medicinala" 1981 pg. 391—Agenda medicala 1981, pg. 666 are known as useful drugs to restore the liver metabolism aiming rather at stopping a rapid evolution of the disease and presenting obvious negative effects.

Also known are compositions used in hepatic and biliary diseases which contain active principal from *Berberis vulgaris* L. alone or associated RO—A—66691, 63127 patents, or vegetable extracts from the plants *Chelidonium majus* L. and *Hypericum perforatum* L. associated with products such as virgin wax, bee larva triturate and propolis powder—RO—A—80826 patent.

The present invention enriches the range of the product intended to treat hepatic and biliary diseases by a herbal composition containing an association of the plants *Epilobium hirsutum* L., *Chamaenerion palustre* schreb. and *Epilobium augustifolium* (L) scop., the respective contents of said plants, calculated on the basis of 100 g of vegetal material, being 15—40 g. 20—50 g and at least 35 g.

Below are given two examples of obtaining the herbal medicinal composition according to the invention.

### Example 1

The composition contains the aerial, underground and subaquatic parts of the plants: 20 g of *Epilobium hirsutum* L., 40 g of *Chamaenerion palustre* schreb., 40 g of *Epilobium augustifolium* (L) scop.

The herbal composition is obtained by mixing the aqueous decoction of the hard parts and roots with the aqueous infusion of their aerial parts.

The aqueous decoction is obtained by boiling the hard parts of the plants in the ratios stated for 7—14 min. The mixture consists of putting 2/3 hard parts and roots with 1/3 leaves, flowers and seeds.

The solution thus obtained is administered 2—3 times/day in an amount of 100—250 g per os/day, 30—45 min. before meals in biliary dyskinesia, chronic hepatic disease and calculus diseases.

### Example 2

The composition consists of the aerial, underground and subaquatic parts of the plants: 10 g of *Epilobium hirsutum* L., 30 g of *Chamaenerion palustre* schreb., 60 g of *Epilobium auqustifolium* (L) scop.

The decoction from the hard and ligneous parts of the plants is made under the same condition as in Example 1, and the other parts of the plants are directly infused in the decoction. The ratios of the hard parts and roots of the plants, in relation to the other parts, are the same as in Example 1. The administration is also the same.

The above composition is intended to treat functional or organic acute and chronic hepatic and biliary diseases, namely biliary diskinesia, calculus biliary diseases, chronic hepatopathies, persistant chronic hepatitis, aggresive chronic hepatitis, compensated biliary cirrhosis, chronic pancreatitis and biliary calculus.

The herbal composition according to the invention has the following advantages:

—has a very good digestive tolerance;

—definitely improves bile secretion, the dynamics of bile elimination into the duodenum, realizing an optimum drainage required by digestion;

—helps significantly to regenerate the liver cells, and increases the detoxifying capacity of the liver;

—improves obviously the immunopathologic processes in chronic hepatic diseases;

—has a strong roborant effect upon the body.

## Claim

Medicinal herbal composition for treating some hepatic and biliary diseases, characterized in that it comprises the plants *Epilobium hirsutum* L., *Chamaenerion palustre* schreb. and *Epilobium augustifolium* (L) scop., the respective contents of said plants, calculated on the basis of 100 g of vegetal matter, being 15—40 g, 20—50 g and at least 35 g.

## Patentanspruch

Medizinische Pflanzen- bzw. Kräuterzusammensetzung zur Behandlung bestimmter Leber- und Gallenerkrankungen, dadurch gekennzeichnet, daß sie die Pflanzen *Epilobium hirsutum* L., *Chamaenerion palustre* schreb, und *Epilobium augustifolium* (L) scop. umfaßt, wobei die entsprechenden Gehalte der Pflanzen, berechnet auf der Basis von 100 g pflanzlichem Material, 15—40 g, 20—50 g und wenigstens 35 g betragen.

## Revendication

Composition médicinale à base de plantes, pour le traitement de certaines maladies biliaires et hépatiques, caractérisée en ce qu'elle comprend les plantes *Epilobium hirsutum* L., *Chamaenerion palustre* schreb. et *Epilobium augustifolium* (L) scop., les teneurs respectives en ces plantes, calculées sur la base de 100 g de matière végétale, étant 15—40 g, 20—50 g et au moins 35 g.